# EUROPÄISCHE PATENTSCHRIFT

(11) **EP 1 053 995 B1**
(45) Veröffentlichungstag und Bekanntmachung des Hinweises auf die Patenterteilung: **08.01.2003**
(21) Anmeldenummer: 00110223.5
(22) Anmeldetag: 18.05.2000
(51) Int. Cl.: C07C 67/327, C07C 67/08, C07C 69/54

(54) **Verfahren zur Herstellung von (Meth)acrylsäureestern**
Process for the preparation of esters of (meth)acrylic acid
Procédé de préparation d'esters de l'acide (méth)acrylique

(30) Priorität: 18.05.1999 DE 19922722
(43) Veröffentlichungstag der Anmeldung: 22.11.2000
(73) Patentinhaber: BASF AKTIENGESELLSCHAFT, 67056 Ludwigshafen (DE)
(72) Erfinder: Dams, Albrecht, Dr., 67157 Wachenheim (DE); Aichinger, Heinrich, Dr., 68199 Mannheim (DE); Herbst, Holger, Dr., 67227 Frankenthal (DE); Nestler, Gerhard, Dr., A-1070 Wien (AT); Schröder, Jürgen, Dr., 67071 Ludwigshafen (DE)
(74) Vertreter: Isenbruck, Günter, Dr.

(56) Entgegenhaltungen:
- EP-A- 0 779 268
- DE-A- 19 701 737

## Beschreibung

Die Erfindung betrifft ein Verfahren zur Herstellung von (Meth)acrylsäureestern, bei dem (Meth)acrylsäure mit Alkanolen in Gegenwart von Veresterungskatalysatoren verestert wird. Nach der Veresterung werden nicht umgesetzte Ausgangsverbindungen und der zu bildende (Meth)acrylsäureester destillativ abgetrennt und ein Oxyester enthaltendes Sumpfprodukt wird erhalten. Neben Oxyestern entstehen auch oligomere (Meth)acrylsäure oder oligomere oder polymere (Meth)acrylsäureester. Diese Verbindungen und ihre Entstehung werden zunächst im folgenden erläutert.

Alkylester der (Meth)acrylsäure sind aufgrund ihrer aktivierten C=C-Doppelbindung wichtige Ausgangsverbindungen zur Herstellung von durch radikalische Polymerisation zu erzeugenden Polymerisaten, die z.B. als Klebstoffe Verwendung finden.

Üblicherweise erfolgt die Herstellung der Ester durch Veresterung der (Meth)acrylsäure mit Alkanolen bei erhöhter Temperatur in flüssiger Phase mit oder ohne Lösungsmittel und in Gegenwart von von (Meth)acrylsäure verschiedenen Säuren als Katalysator und anschließender destillativer Isolierung.

Nachteilig an diesem Verfahren ist, daß einerseits trotz Verwendung von Polymerisationsinhibitoren die Bildung von polymeren (Meth)acrylsäureestern nicht vollkommen verhindert werden kann, und andererseits sich unter den vorgenannten Veresterungsbedingungen als Nebenreaktionen noch nicht umgesetzte Ausgangsalkanole unter Ausbildung einer Verbindung der nachfolgend angeführten allgemeinen Formel I sowie noch nicht umgesetzte (Meth)acrylsäure unter Ausbildung einer Verbindung der allgemeinen Formel II an die Doppelbindung von bereits gebildetem (Meth)acrylsäurealkylester addieren (Michael-Addition).

Auch eine sukzessive Mehrfachaddition ist möglich. Ferner können gemischte Typen auftreten. Diese Addukte (Alkoxyester und Acyloxyester) nennt man kurz Oxyester:

RO(-CH₂-CHR'-CO₂)ₓ-R (I),

CH₂=CR'-CO₂(-CH₂-CHR'-CO₂)_{y}-R (II),

mit
x, y = eine ganze Zahl von 1 bis 5,
R = Alkyl und
R' = H oder CH₃.

Die Entstehung von Oxyestern ist z.B. in der DE-A 23 39 529 beschrieben. Die Bildung von Oxyestern erfolgt im wesentlichen unabhängig von den speziellen Veresterungsbedingungen. Von ganz besonderer Bedeutung ist die Oxyesterbildung bei der Herstellung von Acrylsäureestern.

Die Bezeichnung polymere (Meth)acrylsäureester meint die durch radikalische Polymerisation von (Meth)acrylsäureestern entstehenden polymeren Produkte. Diese Polymerisate sind im Gegensatz zu den Michael-Additionsprodukten unter üblichen Reaktionsbedingungen nicht in die Ausgangsmonomeren rückspaltbar. Sie entstehen bei der thermischen Belastung der (Meth)acrylsäureester, z.B. bei der Synthese oder destillativen Aufarbeitung des Veresterungsgemisches bzw. bei der destillativen Reinigung.

Der Begriff (Meth)acrylsäure bezeichnet Acryl- oder Methacrylsäure.

Die Bezeichnung oligomere (Meth)acrylsäure meint die Michael-Addukte von (Meth)acrylsäure mit sich selbst und den dabei entstehenden Folgeprodukten. Solche Michael-Addukte lassen sich durch die allgemeine Formel (III)

CH₂=CR'-CO₂(-CH₂-CHR'-CO₂)_{z}-H (III),

mit z = eine ganze Zahl von 1 bis 5,
und R' = H oder CH₃,
charakterisieren und sind hier von (monomerer) (Meth)acrylsäure sowie von (Meth)acrylsäurepolymerisaten (die durch radikalische Polymerisation von (Meth)acrylsäure erhältlich sind) zu unterscheiden. Wesentlich ist, daß die Michael-Addition von (Meth)acrylsäure mit sich selbst und den dabei entstehenden Folgeprodukten reversibel ist.

Oligomere (Meth)acrylsäure fällt z.B. bei der destillativen Behandlung von (z.B. roher) (Meth)acrylsäure im Sumpf an (der Begriff "roh" weist einen noch enthaltenen geringen Anteil an insbesondere aldehydischen Verunreinigungen aus).

Die Aufarbeitung eines beliebigen Veresterungs-Reaktionsgemisches erfolgt normalerweise so, daß nicht umgesetzte Ausgangsverbindungen sowie der Zielester vom Reaktionsgemisch destillativ abgetrennt werden, wobei der zur Veresterung verwendete Säurekatalysator und nicht umgesetzte (Meth)acrylsäure gegebenenfalls vorher durch Extraktion mittels Wasser und/oder wäßriger Lauge abgetrennt wird (siehe z.B. Ullmann's Encyclopedia of Industrial Chemistry, Vol. A1, 5th Ed.,VCH S. 167ff). Das im Rahmen einer solchen destillativen Aufarbeitung verbleibende Sumpfprodukt enthält die Oxyester und die polymeren (Meth)acrylsäureester, die einen beträchtlichen Ausbeuteverlust bedingen.

Es wurde daher die Anwendung verschiedenster Verfahrensweisen versucht, um die durch das Auftreten dieser Nebenkomponenten, vor allem der Michael-Additionsprodukte, entstehenden Verluste an Wertprodukten zu mindern.

In der DE-A-195 47 459 und DE-A-195 47 485 sind Verfahren zum Verestern von (Meth)acrylsäure mit einem Alkanol beschrieben, bei dem nach der Veresterungsreaktion aus dem Reaktionsgemisch der gebildete (Meth)acrylsäureester und nicht umgesetzte Ausgangsverbindungen abdestilliert werden und ein Oxyester enthaltendes Sumpfprodukt entsteht. Dem Sumpfprodukt wird entweder unmittelbar (Meth)acrylsäure oder oligomere (Meth)acrylsäure zugesetzt, und anschließend werden die im Sumpfprodukt enthaltenen Oxyester in Anwesenheit von Säurekatalysatoren, die von (Meth)acrylsäure oder oligomerer (Meth)acrylsäure verschieden sind, durch Einwirkung erhöhter Temperatur gespalten, oder die Oxyester werden zunächst aus dem Sumpfprodukt destillativ abgetrennt und das Destillat wird wie vorstehend beschrieben behandelt. Die anfallenden Spaltprodukte werden unmittelbar in die Veresterung zurückgeführt. Der nach der Spaltung und Abtrennung der Spaltprodukte verbleibende Spaltrückstand wird nicht weiter aufgearbeitet.

In der EP-A-0 765 860 ist ein Verfahren zum Verestern von (Meth)acrylsäure mit einem Alkanol beschrieben, bei dem nach der Umsetzung gebildete (Meth)acrylsäureester und nicht umgesetzte Ausgangsverbindungen von einem Oxyester enthaltenen Sumpfprodukt abgetrennt werden. Die im abgetrennten Sumpfprodukt enthaltenen Oxyester werden destillativ abgetrennt und das entstehende Destillat wird in Anwesenheit von Säuren bei erhöhter Temperatur gespalten. Eine Aufarbeitung des Spaltrückstandes ist nicht beschrieben.

In der EP-A-0 767 163 ist ein weiteres Verfahren zum Verestern von (Meth)acrylsäure mit einem Alkanol beschrieben. Nach der Entfernung entstandener (Meth)acrylsäureester und nicht umgesetzter Ausgangsverbindungen wird das Sumpfprodukt abgetrennt und in Anwesenheit von Säure auf 150 bis 200 °C erhitzt, wobei der Druck so eingestellt wird, daß die Spaltprodukte, die bei der Spaltung der im Sumpfprodukt enthaltenen Oxyester entstehen, unmittelbar abdampfen. Eine weitere Behandlung des Spaltrückstandes ist nicht beschrieben.

In der JP-A H8 183 756 ist der säurekatalysierte Abbau von Michael-Additionsprodukten in Gegenwart von Wasser beschrieben. Der Umsatz beträgt jedoch nur etwa 60%.

EP-A-0 779 268 beschreibt ein Verfahren zur Herstellung von Acrylsäurealkylestern, bei dem die hochsiedenden Rückstände (Oxyester) hydrolytisch gespalten werden, das entstehende Alkanol destillativ abgetrennt wird und der Hydrolyserückstand einer Spaltreaktion unterworfen wird.

Das Verfahren hat den Nachteil, daß die Rückgewinnungsrate unbefriedigend ist und daß die im Oxyester enthaltene Acrylsäurealkylesterkomponente hydrolytisch in Alkanol und Acrylsäure gespalten und nicht als solche zurückgewonnen wird.

Aufgabe der vorliegenden Erfindung ist die Bereitstellung eines Verfahrens zum Verestern von (Meth)acrylsäure mit einem Alkanol, bei dem gegenüber den vorstehenden Verfahren der Nebenproduktanteil weiter vermindert wird und aus den verbleibenden Rückständen mehr Produkte gewonnen werden, die in die Veresterung zurückgeführt oder als Zielester ausgeschleust werden können.

Die Aufgabe wird erfindungsgemäß gelöst durch ein Verfahren zum Verestern von (Meth)acrylsäure mit einem Alkanol in Gegenwart eines Veresterungskatalysators, bei dem nicht umgesetzte Ausgangsverbindungen und der zu bildende (Meth)acrylsäureester destillativ abgetrennt werden und ein Oxyester enthaltendes Sumpfprodukt entsteht, das abgetrennt wird, und
(a) in dem abgetrennten Sumpfprodukt, gegebenenfalls nach Versetzen mit (Meth)acrylsäure und/oder oligomerer (Meth)acrylsäure, unmittelbar in Gegenwart von von (Meth)acrylsäure und/oder oligomerer (Meth)acrylsäure verschiedenen Säurekatalysatoren die enthaltenen Oxyester gespalten und die Spaltprodukte entfernt werden, wobei ein Spaltrückstand verbleibt, oder
(b) aus dem abgetrennten Spaltprodukt die Oxyester zunächst destillativ abgetrennt werden, wobei ein Destillationsrückstand verbleibt, und die abgetrennten Oxyester, gegebenenfalls nach Versetzen mit (Meth)acrylsäure und/oder oligomerer (Meth)acrylsäure, in Gegenwart von von (Meth)acrylsäure und/oder oligomerer (Meth)acrylsäure verschiedenen Säurekatalysatoren gespalten und die Spaltprodukte entfernt werden, wobei ein Spaltrückstand verbleibt,
wobei der in Schritt (a) erhaltene Spaltrückstand oder der in Schritt (b) erhaltene Spaltrückstand zusammen mit dem erhaltenen Destillationsrückstand in Gegenwart von Wasser und Säuren oder Basen hydrolytisch gespalten wird.

Der Vorteil des erfindungsgemäßen Verfahrens besteht darin, daß die bei der Herstellung von (Meth)acrylsäureester anfallenden Nebenprodukte, Oxyester und polymere (Meth)acrylsäureester, entsprechend ihrer chemischen Natur gezielt in Wertprodukte, die direkt in die Veresterung zurückgeführt werden können, umgewandelt werden. Es werden hohe Ausbeuten, hohe Spaltgeschwindigkeiten, hochwertige Spaltprodukte (Ester) und geringe und gut handhabbare Rückstandsmengen erhalten. Durch gezielte Spaltung der Oxyester erhält man Ester und Alkanol bzw. Ester und (Meth)acrylsäure. Durch die hydrolytische Spaltung der polymeren Rückstände erhält man Alkanole.

Eine hydrolytische Spaltung des Veresterungssumpfes, der die Oxyester und die polymeren (Meth)acrylsäureester enthält, liefert dagegen hauptsächlich Alkanol und (Meth)acrylsäure bzw. deren Salze, wobei die Isolierung der (Meth)acrylsäure äußerst umständlich ist.

Das erfindungsgemäße Verfahren weist die folgenden Schritte auf:

Zunächst wird auf bekannte Weise ein Veresterungsgemisch durch Verestern von (Meth)acrylsäure mit einem Alkanol in Gegenwart eines Veresterungskatalysators hergestellt.

Bei der Veresterung wird als Alkanol vorzugsweise ein C₁₋₁₂-Alkanol, besonders bevorzugt C₄₋₁₀-Alkanol eingesetzt. Vorzugsweise handelt es sich dabei um Methanol, Ethanol, n-Butanol oder 2-Ethylhexanol, insbesondere um n-Butanol oder 2-Ethylhexanol.

Typische Bedingungen, unter denen die Veresterung stattfinden kann, sind beispielsweise:
Verhältnis Alkohol:(Meth)acrylsäure 1:0,7-1,2 (molar)
Katalysator: Schwefelsäure oder Sulfonsäure (z.B. p-Toluolsulfonsäure)
Katalysatormenge: 0,1 - 10 Gew.-% (vorzugsweise 0,5 - 5 Gew.-% bezüglich Einsatzstoffe)
Stabilisierung: 200 - 2000 ppm Phenothiazin (bezogen auf das Gewicht der Einsatzstoffe)
Reaktionstemperatur: 80 - 160 °C, vorzugsweise 90 - 130 °C
Reaktionszeit: 1 - 10 Std., vorzugsweise 1 - 6 Std.

Gegebenenfalls wird ein Schleppmittel (z.B. Cyclohexan oder Toluol) zur Entfernung des Veresterungswassers eingesetzt. Die Veresterung kann drucklos, mit Überdruck oder Unterdruck und sowohl kontinuierlich als auch diskontinuierlich durchgeführt werden.

Nach der Umsetzung wird der Veresterungskatalysator, beispielsweise durch Behandlung des Reaktionsgemisches mit Wasser, aus dem Reaktionsgemisch entfernt. Der gebildete (Meth)acrylsäureester und die nicht umgesetzten Ausgangsverbindungen werden destillativ abgetrennt.

Bei der säurekatalysierten Veresterung von Acrylsäure mit Alkanolen hat das nach der Abtrennung des sauren Veresterungskatalysators, der nicht umgesetzten Ausgangsstoffe und des Acrylesters resultierende Sumpfprodukt in der Regel folgende Zusammensetzung:
1 - 20 Gew.-% Acrylester
50 - 80 Gew.-% Alkoxypropionate (siehe allgemeine Formel I)
3 - 30 Gew.-% Acryloxypropionate (siehe allgemeine Formel II)
5 - 20 Gew.-% Polymere der Acrylester
Rest: Hauptsächlich Inhibitoren

Die im Sumpfprodukt enthaltenen Oxyester werden sodann entweder direkt im Sumpfprodukt oder nach destillativer Abtrennung aus dem Sumpfprodukt gespalten.

Die direkte säurekatalysierte Oxyesterspaltung im Sumpfprodukt ist beispielsweise in EP-A-0 767 163 beschrieben. Die destillative Abtrennung der Oxyester und nachfolgende Spaltung ist beispielsweise in EP-A-0 765 860 beschrieben. Die Spaltung der Oxyester in Gegenwart von monomerer und/oder oligomerer (Meth)acrylsäure ist beispielsweise in DE-A-195 47 485 und DE-A-195 47 459 beschrieben.

Typische Bedingungen für die Durchführung des Verfahrens der Spaltung der bei der Veresterung im Sumpfprodukt anfallenden bzw. vom Sumpfprodukt abgetrennten Oxyester sind folgende:
- Katalysator:: wenigstens eine Säure aus der Gruppe der Mineralsäuren, wie Schwefelsäure und Phosphorsäure, sowie von oligomerer (Meth)acrylsäure verschiedener organischer gegebenenfalls wäßriger Säuren, wie Alkyl- oder Arylsulfonsäuren, z.B. Dodecylsulfonsäure oder p-Toluolsulfonsäure
- Katalysatormenge:: 1-20 Gew.-%, vorzugsweise 5 - 15 Gew.-%, bezogen auf die Menge des Sumpfprodukts bzw. auf die Menge des vom Sumpfprodukt abgetrennten Oxyesterdestillats
- Menge an monomerer und/oder oligomerer (Meth)acrylsäure:: 5 - 50 Gew.-%, vorzugsweise 10 - 40 Gew.-%, bezogen auf die Menge des Sumpfprodukts bzw. auf die Menge des vom Sumpfprodukt abgetrennten Oxyesterdestillats
- Temperatur:: 150 - 250°C, vorzugsweise 180 - 230°C
- Druck:: vorzugsweise drucklos bzw. verminderter Druck (< 1 atm), so daß die Spaltprodukte unmittelbar abdampfen
- ggf. Strippgas:: Menge: 1 - 100 l/h x 1 (vorzugsweise Sauerstoff enthaltendes Gas)
- Reaktionszeit:: 1 - 10 Std.

Für die Spaltung der bei der Veresterung im Sumpfprodukt anfallenden Oxyester bzw. des vom Veresterungssumpf abgetrennten Oxyesterdestillats kann ein einfacher beheizbarer Rührreaktor mit Doppelwandheizung oder Heizschlange oder auch ein Zwangsumlaufverdampfer, beispielsweise ein Fallfilmverdampfer oder Flachverdampfer, gekoppelt mit einem Verweilzeitbehälter, verwendet werden. Zur besseren Trennung der Spaltprodukte vom Sumpfprodukt bzw. Oxyesterdestillat ist gegebenenfalls eine auf die Spaltapparatur aufgesetzte Rektifikationsvorrichtung, z.B. eine Füllkörper-, Pakungs- oder Bodenkolonne zweckmäßig. Diese Rektifikationsvorrichtung wird in der Regel mit Polymerisationinhibitoren (z.B. Phenothiazin, Hyrochinonmonomethylether etc.) stabilisiert.

Die Reaktionsführung läuft z.B. in der Weise, daß das zu spaltende Produkt kontinuierlich aus der destillativen Aufarbeitung des Veresterungsgemisches ausgeschleust und mit dem Spaltkatalysator und gegebenenfalls der monomeren und/oder oligomeren (Meth)acrylsäure dem Spaltreaktor zugeführt wird. Die Reaktionsführung kann aber auch diskontinuierlich, d.h. batchweise, durchgeführt werden. Auch eine halbkontinuierliche Reaktionsführung ist möglich, bei der das zu spaltende Produkt, und gegebenenfalls die monomere und/oder oligomere (Meth)acrylsäure kontinuierlich dem Spaltreaktor, der den Spaltkatalysator enthält, zugeführt werden und das Sumpfprodukt erst nach Beendigung der Spaltung aus dem Spaltreaktor batchweise entfernt wird. Die Spaltprodukte werden kontinuierlich destillativ abgetrennt und zweckmäßigerweise in die Veresterung rückgeführt.

Als günstig erweist es sich, wenn durch das zu spaltende Produkt als Schleppmittel für die Spaltprodukte ein Strippgas, das vorzugsweise molekularen Sauerstoff enthält, geführt wird. Zweckmäßigerweise werden als Strippgas Luft oder Gemische von Luft mit Inertgas (z.B. Stickstoff) verwendet

Bei der destillativen Abtrennung der Oxyester vom Sumpfprodukt richten sich die Destillationsbedingungen nach der Art der bei der Veresterung eingesetzten Alkanolkomponente. In der Regel sind eine Temperatur von 100 bis 300°C und ein Druck von 1 bis 50 mbar vorgesehen. Für das Destillationsverfahren eignet sich jede herkömmliche Destillationsapparatur. Da nur eine einfache Trennaufgabe zu lösen ist, genügt in der Regel ein einfacher Spritzschutz, d.h. eine Kolonne ist normalerweise nicht erforderlich.

Nach der Abtrennung der bei der Spaltung entstehenden Spaltprodukte verbleibt ein Spaltrückstand. Werden zunächst die Oxyester aus dem bei der Veresterung entstehenden Sumpfprodukt destillativ abgetrennt, so verbleibt zudem ein Destillationsrückstand. Diese, gegebenenfalls vereinigten, Rückstände werden sodann in Gegenwart von Wasser und Säuren oder Basen hydrolytisch gespalten.

Bevorzugte Hydrolysebedingungen sind wie folgt:

Die Hydrolyse kann drucklos, d.h. bei Umgebungsdruck, bei Unterdruck oder Überdruck durchgeführt werden. Die Hydrolyse der Rückstände kann in einer der Spaltung analogen Apparatur durchgeführt werden. Sie kann sowohl kontinuierlich als auch diskontinuierlich durchgeführt werden. Vorzugsweise wird sie diskontinuierlich in einem Rührreaktor mit Doppelwandheizung durchgeführt.

Wird die Hydrolyse säurekatalysiert durchgeführt, so genügt in der Regel die im Spaltrückstand enthaltene starke Säure. Zur Erhöhung der Reaktionsgeschwindigkeit kann aber die Zugabe weiterer Säuren, z.B. Mineralsäuren, wie Schwefelsäure oder Phosphorsäure, p-Toluolsulfonsäure, oder anderer organischer Säuren, wie sie vorstehend beschrieben sind, sinnvoll sein (1 - 20 Gew.-%).

Wird die Hydrolyse durch Basen katalysiert, vorzugsweise Alkali- und Erdalkalilaugen, so ist die Menge des vorhandenen sauren Spaltkatalysators zu berücksichtigen. In der Regel genügen 10 - 50 Gew.-% Base, bezogen auf die Rückstände.

Die am Anfang in der Regel zuzusetzende Wassermenge beträgt 20 - 200, vorzugsweise 50 - 150 %, bezogen auf die organische Phase, unabhängig von der Art des Hydrolysekatalysators.

Die Hydrolyse wird z.B. in der Weise durchgeführt, daß die Rückstände, der Katalysator und das Wasser in einem Rührreaktor vorgelegt und zum Sieden erhitzt werden, wobei die in der Spaltung entstehenden Hydrolyseprodukte und ein Teil des Wassers abdestilliert werden. Das dabei anfallende Destillat zerfällt in eine Wasserphase, die nach einer Phasentrennung wieder der Hydrolyse zugeführt wird, und eine organische Phase, die hauptsächlich aus dem entsprechenden Alkanol besteht. Die organische Phase wird vorzugsweise wieder direkt in die Veresterung bzw. dem Veresterungsreaktor zugeführt.

Bei der hydrolytischen Spaltung kann ein Strippgas, wie es vorstehend beschrieben ist, durch den Spaltrückstand und gegebenenfalls Destillationsrückstand geführt werden.

Die Erfindung wird nachstehend anhand von Beispielen näher erläutert.

### Beispiel 1

### (A) Destillation der Oxyester

Eine Destillationsapparatur aus einem Rundkolben (61), einer aufgesetzten Kolonne (30 cm x 2,8 cm; Raschigringe 5 mm) und einem Kondensator wurde mit 4 kg einer bei der Herstellung von Butylacrylat anfallenden, keinen sauren Veresterungskatalysators mehr aufweisenden Sumpfflüssigkeit folgender Zusammensetzung gefüllt:
7,4 Gew.-% Butylacrylat
64,5 Gew.-% Butoxyester I(R = C₄H₉)
19,2 Gew.-% Acyloxyester II (R = C₄H₉)
Rest: hauptsächlich Polymere und Phenothiazin (Polymerisationsinhibitor)

Die Destillation erfolgte bei einem Druck vom 30 mbar bis zu einer Temperatur von 150°C. Das anfallende Destillat (90% der Einsatzmenge) enthielt entsprechend der gaschromatographischen Anlayse:
8,5 Gew.-% Butylacrylat
68,3 Gew.-% Butoxyester I (R = C₄H₉)
20,0 Gew.-% Acyloxyester II (R = C₄H₉)

Eine Stabilisierung der Kolonne mit Phenothiazin oder einer anderen üblichen Stabilisierung war nicht notwendig. Der anfallende Destillationssumpf war bei 25°C noch gut handhabbar (pumpfähig) und enthielt keine Feststoffe.

### (B) Spaltung der Oxyester

Ein aus Glas bestehender Umlaufreaktor (Volumen 11), beheizt mit einer Heizkerze, wurde mit 500 g des Oxyesterdestillats aus (A) gefüllt, wobei zusätzlich 40 g p-Toluolsulfonsäure und 150 g Acrylsäure (mit 300 ppm Phenothiazin stabilisiert) zugesetzt wurden.

Die Spalttemperatur betrug 195°C, der Arbeitsdruck betrug 1 atm.

Standgeregelt wurden dem Spaltreaktor das zu spaltende Oxyesterdestillat aus (A) und der entsprechender Acrylsäurezusatz (30 Gew.-%) kontinuierlich zugeführt. Die Spaltprodukte wurden am Kopf der auf dem Reaktor aufgesetzten Kolonne (50 cm x 2,8 cm, leer) kondensiert.

Pro Stunde wurde 100 g Oxyesterdestillat und 30 g stabilisierte Acrylsäure der Spaltung zugeführt und 125,5 g Kondensat gewonnen. Entsprechend der gaschromatographischen Analyse enthielt das Kondensat:
71,8 Gew.-% Butylacrylat
5,1 Gew.-% Butanol
18,2 Gew.-% Acrylsäure
0,5 Gew.-% Dibutylether
1,8 Gew.-% Butene
Umsatz: ca. 96,5 %, bezogen auf Oxyester

Das entsprach ca. 810 g Butylacrylat und ca. 58 g Butanol pro 1000 g Veresterungssumpf.

### (C) Hydrolyse des Spaltrückstandes und Destillationssumpfes

In einem aus Glas bestehenden Rührreaktor (21) mit aufgesetzter Kolonne (60 cm x 2,8 cm; Raschigringe 5 mm) und Kondensator wurde ein Gemisch aus 400 g Destillationssumpf aus (A) und 195 g Spaltrückstand (B) und 600 g 10 %-ige Schwefelsäure unter Rühren zum Sieden erhitzt und Wasser und Hydrolyseprodukte kontinuierlich abdestilliert. Die wäßrige Phase des Kondensats wurde dem Reaktor wieder zugeführt (Rücklauf) und die organische Phase isoliert (214 g). Diese enthielt entsprechend der gaschromatographischen Analyse 28,6 % Butylacrylat und 52,6 % Butanol.
In Summe (Spaltung + Hydrolyse) erhielt man aus 1000 g Veresterungssumpf ca. 825 g Butylacrylat und ca. 86 g Butanol.

### Beispiel 2 (Vergleichsbeispiel)

In der unter Beispiel 1 (C) beschriebenen Apparatur wurden 600 g der unter Beispiel 1 (A) eingesetzten Sumpfflüssigkeit in Gegenwart von 20 g Schwefelsäure analog (C) hydrolysiert. Es fielen 415 g organische Phase an, die entsprechend der gaschromatographischen Analyse 7,7 % Butylacrylat und 75,7 % Butanol enthielt.
Es konnten demnach pro 1000 g Veresterungssumpf nur ca. 53 g Butylacrylat und ca. 523 g Butanol zurückgewonnen werden.

### Beispiel 3

### (A) Spaltung des Veresterungsrückstandes

Ein aus Glas bestehender Umlaufreaktor (Volumen: 11), beheizt mit einer Heizkerze, wurde mit 500 g eines vom sauren Veresterungskatalysator befreiten Veresterungsrückstand der n-Butylacrylatherstellung, 150 g Acrylsäure (mit 300 ppm Phenothiazin stabilisiert) und 50 g p-Toluolsulfonsäure gefüllt.

Der Veresterungsrückstand enthielt
7,4 Gew.-% Butylacrylat,
64,5 Gew.-% Butoxyester I (R = C₄H₉)
19,2 Gew.-% Acryloxyester II (R = C₄H₉)

Die Spalttemperatur betrug 195°C, und der Arbeitsdruck lag bei 1 atm.

Standgeregelt wurde dem Spaltreaktor während der Spaltung kontinuierlich der zu spaltende Veresterungsrückstand und die entsprechende Acrylsäuremenge zugeführt.

Die Spaltprodukte wurden dampfförmig abgeführt und am Kopf der auf dem Spaltreaktor aufgesetzten Kolonne (30 cm X 2,8 cm, 5 mm Raschigringe) kondensiert.

Pro Stunde wurden 80 g Veresterungsrückstand und 24 g Acrylsäure der Spaltung zugeführt und 95 g Destillat kondensiert.

Entsprechend der gaschromatographischen Analyse enthielt das Kondensat:
71,0 Gew.-% Butylacrylat
6,5 Gew.-% Butanol
18,5 Gew.-% Acrylsäure
0,4 Gew.-% Dibutylether
1,0 Gew.-% Butene
Umsatz: 90 Gew.-%, bezogen auf den Veresterungsrückstand.

### (B) Hydrolyse des Spaltrückstandes

In einem Rührreaktor gemäß Beispiel 1C wurde ein Gemisch aus 524 g Spaltrückstand und 780 g 25 %-iger Natronlauge zum Sieden erhitzt, und die Hydrolyseprodukte wurden destillativ abgetrennt. Die Wasserphase wurde in den Reaktor zurückgeführt. Die Temperatur im Reaktor betrug anfangs 97°C und stieg innerhalb von 4 Stunden auf 103°C an.
Die organische Phase (208 g) enthielt entsprechend der gaschromatographischen
Analyse vor allem Butanol (78 %) und Wasser (17 %).

In Summe konnten demnach aus 1000 g Veresterungssumpf ca. 104 g Butanol und ca. 843 g Butylacrylat zurückgewonnen werden.

## Patentansprüche

1. Verfahren zum Verestern von (Meth)acrylsäure mit einem Alkanol in Gegenwart eines Veresterungskatalysators, bei dem nicht umgesetzte Ausgangsverbindungen und der zu bildende (Meth)acrylsäureester destillativ abgetrennt werden und ein Oxyester enthaltendes Sumpfprodukt entsteht, das abgetrennt wird, und
(a) in dem abgetrennten Sumpfprodukt, gegebenenfalls nach Versetzen mit (Meth)acrylsäure und/oder oligomerer (Meth)acrylsäure, unmittelbar in Gegenwart von von (Meth)acrylsäure und/oder oligomerer (Meth)acrylsäure verschiedenen Säurekatalysatoren die enthaltenen Oxyester gespalten und die Spaltprodukte entfernt werden, wobei ein Spaltrückstand verbleibt, oder
(b) aus dem abgetrennten Sumpfprodukt die Oxyester zunächst destillativ abgetrennt werden, wobei ein Destillationsrückstand verbleibt, und die abgetrennten Oxyester, gegebenenfalls nach Versetzen mit (Meth)acrylsäure und/oder oligomerer (Meth)acrylsäure, in Gegenwart von von (Meth)acrylsäure und/oder oligomerer (Meth)acrylsäure verschiedenen Säurekatalysatoren gespalten und die Spaltprodukte entfernt werden, wobei ein Spaltrückstand verbleibt,
**dadurch gekennzeichnet, daß** der in Schritt (a) erhaltene Spaltrückstand oder der in Schritt (b) erhaltene Spaltrückstand zusammen mit dem erhaltenen Destillationsrückstand in Gegenwart von Wasser und Säuren oder Basen hydrolytisch gespalten wird.

2. Verfahren nach Anspruch 1, **dadurch gekennzeichnet, daß** die Spaltung in Schritt (a) oder (b) bei einer Temperatur im Bereich von 150 bis 250°C und die hydrolytische Spaltung bei einer Temperatur im Bereich von 80 bis 120°C durchgeführt wird.

3. Verfahren nach Anspruch 1 oder 2, **dadurch gekennzeichnet, daß** die bei der Spaltung der Oxyester entstehenden Spaltprodukte direkt in die Veresterung zurückgeführt werden.

4. Verfahren nach einem der Ansprüche 1 bis 3, **dadurch gekennzeichnet, daß** die bei der hydrolytischen Spaltung entstehenden Hydrolyseprodukte mit einem Teil des Wassers abdestilliert werden und nach einer Phasentrennung in eine wäßrige und eine organische Phase die wäßrige Phase in die hydrolytische Spaltung und die organische Phase in die Veresterung zurückgeführt wird.

5. Verfahren nach einem der Ansprüche 1 bis 4, **dadurch gekennzeichnet, daß** als Säurekatalysator in den Schritten (a) und (b) Mineralsäuren oder von (Meth)acrylsäure und oligomerer (Meth)acrylsäure verschiedene organische Säuren eingesetzt werden.

6. Verfahren nach einem der Ansprüche 1 bis 5, **dadurch gekennzeichnet, daß** zur hydrolytischen Spaltung als Säure Mineralsäuren oder von (Meth)acrylsäure und oligomerer (Meth)acrylsäure verschiedene organische Säuren oder im Spaltrückstand vorliegende Säuren und als Base Alkali- oder Erdalkalihydroxide eingesetzt werden.

7. Verfahren nach einem der Ansprüche 1 bis 6, **dadurch gekennzeichnet, daß** zur hydrolytischen Spaltung Wasser in einer Menge von 20 bis 100 Gew.-%, bezogen auf die Menge an Spaltrückstand und gegebenenfalls Destillationsrückstand, zugesetzt wird.

8. Verfahren nach einem der Ansprüche 1 bis 7, **dadurch gekennzeichnet, daß** die Spaltung in den Schritten (a) und (b) unter vermindertem Druck (<1 atm) durchgeführt wird.

9. Verfahren nach einem der Ansprüche 1 bis 8, **dadurch gekennzeichnet, daß** als Alkanol ein C₁₋₁₂-Alkanol eingesetzt wird.

10. Verfahren nach einem der Ansprüche 1 bis 9, **dadurch gekennzeichnet, daß** zur Entfernung der Spaltprodukte in den Schritten (a) und (b) oder bei der hydrolytischen Spaltung ein Strippgas durch den Spaltrückstand und gegebenenfalls Destillationsrückstand geführt wird.

## Claims

1. A process for the esterification of (meth)acrylic acid with an alkanol in the presence of an esterification catalyst, in which unconverted starting compounds and the (meth)acrylate to be formed are separated off by distillation and an oxyester-containing bottom product is formed and is separated off, and
(a) in the bottom product separated off, the oxyesters present are cleaved, if required after the addition of (meth)acrylic acid and/or oligomeric (meth)acrylic acid, directly in the presence of acid catalysts differing from (meth)acrylic acid and/or oligomeric (meth)acrylic acid, and the cleavage products are removed, a cleavage residue remaining, or
(b) the oxyesters are first removed by distillation from the cleavage product separated off, a distillation residue remaining, and the oxyesters removed are cleaved, if required after addition of (meth)acrylic acid and/or oligomeric (meth)acrylic acid, in the presence of acid catalysts differing from (meth)acrylic acid and/or oligomeric (meth)acrylic acid, and the cleavage products are removed, a cleavage residue remaining,
wherein the cleavage residue obtained in step (a) or the cleavage residue obtained in step (b) is hydrolytically cleaved together with the resulting distillation residue in the presence of water and acids or bases.

2. A process as claimed in claim 1, wherein the cleavage in step (a) or (b) is carried out at from 150 to 250°C and the hydrolytic cleavage is carried out at from 80 to 120°C.

3. A process as claimed in claim 1 or 2, wherein the cleavage products formed in the cleavage of the oxyesters are recycled directly to the esterification.

4. A process as claimed in any of claims 1 to 3, wherein the hydrolysis products formed in the hydrolytic cleavage are distilled off with a part of the water and, after phase separation into an aqueous and an organic phase, the aqueous phase is recycled to the hydrolytic cleavage and the organic phase to the esterification.

5. A process as claimed in any of claims 1 to 4, wherein the acid catalyst used in steps (a) and (b) is a mineral acid or an organic acid differing from (meth)acrylic acid and oligomeric (meth)acrylic acid.

6. A process as claimed in any of claims 1 to 5, wherein the acid used for the hydrolytic cleavage is a mineral acid or an organic acid differing from (meth)acrylic acid and oligomeric (meth)acrylic acid or is an acid present in the cleavage residue, and the base used is an alkali metal hydroxide or alkaline earth metal hydroxide.

7. A process as claimed in any of claims 1 to 6, wherein water in an amount from 20 to 100% by weight, based on the amount of cleavage residue and any distillation residue, is added to the hydrolytic cleavage.

8. A process as claimed in any of claims 1 to 7, wherein the cleavage in steps (a) and (b) is carried out under reduced pressure (< 1 atm).

9. A process as claimed in any of claims 1 to 8, wherein the alkanol used is a C₁₋₁₂-alkanol.

10. A process as claimed in any of claims 1 to 9, wherein a stripping gas is passed through the cleavage residue and any distillation residue in order to remove the cleavage products in steps (a) and (b) or in the hydrolytic cleavage.

## Revendications

1. Procédé d'estérification d'acide (méth)acrylique par un alcanol en présence d'un catalyseur d'estérification, dans lequel les composés de départ et les esters d'acide (méth)acrylique à former sont séparés par distillation et il se forme une fraction de queue contenant des oxyesters, qui est séparé, et
(a) dans la fraction de queue séparée, éventuellement après mélange avec de l'acide (méth)acrylique et / ou de l'acide (méth)acrylique oligomère, directement en présence de catalyseurs acides différents de l'acide (méth)acrylique et / ou de l'acide (méth)acrylique oligomère, les oxyesters obtenus sont décomposés et les produits de décomposition sont éliminés, avec subsistance d'un résidu de décomposition, ou bien
(b) les oxyesters sont d'abord séparés par distillation de la fraction de queue séparée, avec subsistance d'un résidu de distillation, et les oxyesters séparés, éventuellement après mélange avec de l'acide (méth)acrylique et / ou de l'acide (méth)acrylique oligomère, en présence de catalyseurs acides différents de l'acide (méth)acrylique et / ou de l'acide (méth)acrylique oligomère, sont décomposés, et les produits de décomposition sont éliminés, avec subsistance d'un résidu de décomposition,
**caractérisé en ce que** le résidu de décomposition obtenu à l'étape (a) ou le résidu de décomposition obtenu à l'étape (b) est décomposé par hydrolyse en même temps que le résidu de distillation obtenu, en présence d'eau et d'acides ou de bases.

2. Procédé selon la revendication 1, **caractérisé en ce que** la décomposition dans l'étape (a) ou (b) est entreprise à une température de l'ordre de 80 à 120°C.

3. Procédé selon la revendication 1 ou 2, **caractérisé en ce que** les produits de décomposition formés par la décomposition des oxyesters sont directement recyclés dans l'estérification.

4. Procédé selon l'une des revendications 1 à 3, **caractérisé en ce que** les produits d'hydrolyse formés par la décomposition électrolytique sont éliminés par distillation avec une partie de l'eau et, après une séparation de phases en une phase aqueuse et une phase organique, la phase aqueuse est recyclée dans la décomposition hydrolytique et la phase organique dans l'estérification.

5. Procédé selon l'une des revendications 1 à 4, **caractérisé en ce que** l'on met en oeuvre comme catalyseur acide dans les étapes (a) et (b) des acides minéraux ou des acides organiques différents de l'acide (méth)acrylique et de l'acide (méth)acrylique oligomère.

6. Procédé selon l'une des revendications 1 à 5, **caractérisé en ce que** l'on met en oeuvre pour la décomposition hydrolytique, en tant qu'acides, des acides minéraux ou des acides organiques différents de l'acide (méth)acrylique et de l'acide (méth)acrylique oligomère ou des acides présents dans le résidu de décomposition et, comme bases, des hydroxydes de métaux alcalins ou alcalino-terreux.

7. Procédé selon l'une des revendications 1 à 6, **caractérisé en ce que** l'on ajoute, pour la décomposition hydrolytique, de l'eau en une quantité de 20 à 100% en poids, par rapport à la quantité de résidu de décomposition et éventuellement de résidu de distillation.

8. Procédé selon l'une des revendications 1 à 7, **caractérisé en ce que** la décomposition est entreprise dans les étapes (a) et (b) sous pression réduite (< 1 atm).

9. Procédé selon l'une des revendications 1 à 8, **caractérisé en ce que** l'on met en oeuvre en tant qu'alcanol un alcanol en C₁ à C₁₂.

10. Procédé selon l'une des revendications 1 à 9, **caractérisé en ce que**, pour éliminer les produits de décomposition dans les étapes (a) et (b) ou lors de la décomposition hydrolytique, on fait passer un gaz de stripage dans le résidu de décomposition et éventuellement le résidu de distillation.
